# EUROPEAN PATENT APPLICATION

(11) **EP 0 813 877 A2**
(43) Date of publication of application: **29.12.1997**
(21) Application number: 97304212.0
(22) Date of filing: 16.06.1997
(51) Int. Cl.: A61K 38/18, A61K 31/68, A61K 31/375

(54) **Method for treating congestive heart failure**

(30) Priority: 17.06.1996 US 20276 P
(71) Applicant: SMITHKLINE BEECHAM CORPORATION, Philadelphia Pennsylvania 19103 (US)
(72) Inventor: Wong, Bruce, Philadelphia, Pennsylvania 19103 (US)
(74) Representative: Denholm, Anna Marie

(57) **Abstract**

This invention covers a method for treating congestive heart failure (CHF) by treating or preventing anemia in a patient suffering from CHF.

## Description

### Scope of the Invention

This invention covers a method for treating congestive heart failure (CHF). More specifically, this invention relates to a method for treating CHF by prophylactic treatment of anemia in a patient suffering from CHF, or by treating anemia in a CHF patient who is experiencing poor performance. Also, this invention relates to a means for reducing adverse cost outcomes in CHF patients; this end is achieved by treating the anemia before it reaches a stage where the CHF patient is adversely affected and requires more costly medical intervention.

### Area of the Invention

Congestive heart failure (CHF) is the symptomatic description of end organ failure of the heart muscle to act as a pump. The failure of the heart to pump blood leads to accumulation of fluid or congestion in the body areas from which the blood was to be pumped. In the case of right ventricular failure the consequence is systemic edema often manifest initially by symptomatic dependent edema at peripheral sites (e.g. ankles). In the case of left heart failure (LHF) the fluid accumulation occurs in the lungs and is manifest by pulmonary edema the primary symptom of which is shortncss of breath. Left heart failure is more common and important because of its' relative size and the physiologic function of providing systemic circulation to critical organs.

Traditionally, treatment of chronic mild failure has included limitation of physical activity, restriction of salt intake, and the use of a diuretic. If these measures are not sufficient, digoxin, which is an agent that increases the force of mycardial contraction, is typically added to the treatment regiment. Subsequently, angiotensin converting enzyme inhibitors, which are compounds that prevent the conversion of angiotensin I into the pressor-active angiotensin II, are prescribed for chronic treatment of congestive heart failure, in conjunction with a diuretic, digoxin, or both.

Congestive heart failure occurs as a result of impaired pumping capability of the heart and is associated with activation of both the renin-angiotension system (RAS) and the sympathetic nervous system (SNS). Modulation of the RAS by angiotensin converting enzyme inhibitors has been shown to improve the symptoms associated with CHF. Sharpe, D.N., Murphy, J., Coxon, R. & Hannan S.F. (1984) *Circulation,* **70**, 271-278. However, ACE inhibitors appear to have little effect on the enhanced SNS in CHF. Cohn, J.N., Johnson, G. & Ziesche, S., (1991) *N. Engl. J*. *Med.,* **325**, 293-302 and Francis, G.S., Rector, T.S. & Cohn, J.N. (1988) *Am*. *Heart J.,* **116**, 1464-1468.

The primary underlying cause of heart failure (low output failure) is coronary disease leading to myocardial infarction and cardiac muscle death. Additional other pathologies include cardiomyopathies and myocarditis, valvular dysfunction, metabolic and endocrine abnormalities (e.g. hypothyroidism, alcohol, Mg deficiency etc.) and pericardial abnormalities.

In 1992, CHF prevalence was estimated to be approximately 1% of the general population, 6.8% of patients over the age of 65, and 0.2% - 0.4% of the managed care population (prior to the active recruiting of senior members).2,3,4. In absolute numbers this represents approximately 2.76 million patients in the USA. Subclassifcation by severity provides estimates of 10% as severe, 40% moderate, and the remainder mild. New cases of diagnosed CHF are expected to increase 29%, from 495,700 per year in 1992 to 642,000 by the year 2007. This increase can be attributed to several factors, including the general aging of the population, as well as improvements in physician awareness and diagnostic techniques.

Treatment of CHF is primarily symptomatic through three pathophysiologic mechanisms:-

Afterload reduction - Reduction of the resistance against which the heart must pump blood. Primarily achieved pharmacologically although in severe situations aortic counter pulsation is used.

Cardiac Inotropic state - Improvement of the efficiency of the heart through pharmacological agents acting directly on myocardium (e.g. cardiac glycosides), reduction in starling equilibrium's through chamber size reduction and improvement in coronary artery blood flow.

Pre-load reduction - Reduction in the volume of blood entering the heart. This can be achieved through pharmacological interventions (diuretics, vasodilators), dietary (salt and water restriction) and physical means (rotating tourniquets, blood volume reduction).

The primary pharmacological interventions, therefore, consist of agents from three primary classes, diuretics, cardiac glycosides and ACE inhibitors. In addition recent studies have shown benefit from the use of beta-blockers with anti-oxidant properties. Ultimately cardiac transplantation now offers an alternative to terminal life threatening Heart failure.

A new means for treating CHF has been discovered. Treating, even more appropriately preventing, anemia can prevent the onset of a CHF event or reduce its severity.

### Summary Of the Invention

In one aspect this invention covers treating CHF by the phrophylatic treatment of anemia in a patient suffering from CHF by raising the RBC of said patient by appropriate means associated with the treating the underlying cause of the anemia.

In another aspect this invention relates to the treatment of CHF by treating the anemic patient suffering from morbidity associated with CHF by raising the patient's RBC by appropriate means associated with the treating the underlying cause of the anemia.

In a third aspect this invention relates to a method for providing preventive care to a CHF patient and thus reducing the expense of medical care, this method comprising treating such a patient in a manner which prevents the onset or worsening of anemia. This involves treating said patient prophylactically with a drug such as erythropoietin (EPO) to increase the red blood cell count, or infusing said patient with red blood cells at periodic intervals.

In a further aspect this invention relates to a method for preventing the onset of symptoms in a patient suffering CHF which method comprises maintaining the RBC in about the normal range for said patient. Also it includes a method for treating the symptoms of CHF in a patient suffering CHF which method comprises increasing the RBC to about the normal range for said patient.

### Detailed Description of the Invention

Anemia has been found to correlate to increased cost of care in CHF patients. Statistical analyses show that the possibility of anemia being associated with CHF hospitalization is p = 9 x 10⁻⁴⁵ based on a Chi-square analysis. This p value is considered to be significant; that is if a CHF patient is not treated for anemia medical intervention, and costly medical intervention, will become necessary. Secondarily once anemia develops if it is not treated the effect will be that of greater disability and greater cost in patient treatment.

In regards to how anemia affects CHF, it reduces diastolic function. It is also associated with decreased myocardial and tissue perfusion. It has been reported that end-stage renal patients who develop anemia may also spontaneously develop congestive heart failure, notwithstanding they may not have had active CHF prior to the renal failure. This same study reported that patients who already have CHF and develop renal failure seemingly experience a greater recurrence of CHF phenomena as compared with CHF patients who do not have renal failure. It has also been reported that LVH and left ventricle dilation in end-stage renal failure can result in symptomatic CHF.¹ However, others have reported that anemia does not lead to congestive heart failure in the absence of underlying heart disease.²
¹ See Staffeld CG, Pastan SO., Cardiology Clinics, 13(2):209-23, 1995 May.
² Harnett, J. D. Et al, A. J. Kid Dis., Vol 25, No. 4, Suppl (April), 1995: pp S3-S7. But see Bah., V.K. et al, Am. Heart J., Vol 124, No. 6, (Dec.) 1992, pp 1516-1523.

In any event, it now has been found that anemia is predictive of future hospitalization for CHF patients. And the cost of this hospitalization may be quite high as compared with other benchmarks for increased calls on medical services by people suffering CHF.

Anemia is a decrease in the number of red blood cells (RBC) or hemoglobin (Hb) content due to blood loss, impaired production, increased RBC destruction, or a combination of two or more of these factors.

The normal range at sea level for a RBC is 5.4 million/microL (±0.8) for men and 4.8 million/microL (±0.6) for women.

The normal Hb level for men is 16 (±12) gm/dL and 14 (±2) gm/dL for women. The hematocrit, the volume of RBCs, is 47% (±5) for men and 42% (±5) for women.

The diagnostic criteria for anemia in men are an RBC <4.5 million/microrL, a Hb <14 gm/dL or a Hct <42%; for women these criteria are an RBC <4.5 million/microL, Hb <12 gm/dL, or a Hct <37%.

Anemia may be caused by any one or more of a number of events or physiological failings. A list of possible causes can be found in standard medical texts. For example The Merck Manual, Sixteenth Edition, [published by Merck Research Laboratories, Division of Merck & Co., Inc., Rahway, NJ, USA) lists the following categories and sub-categories of causes of anemia.
- Blood loss:. acute or chronic.
- Deficient Erythropoiesis
   * With hypochromic-microcytic RBC's 1. iron deficiency; 2. iron-transport deficiency; 3. iron-utilization disorders; 4. iron reutilization in chronic disease; 5. thalassemias.
   * With normochronic-normocytic RBC's: 1. hypoproliferation, a. In renal disease, b. In endocrine failure (thyroid, pituitary), c. In protein depletion; 2. Hypoplasia (aplastic anemias); 3. Myelophthisis; and 4. Myelodysplasia.
   * With megaloblastic RBC's: 1. Vitamin B₁₂ deficiency; 2. Folic acid deficiency; 3. Copper deficiency; and 4. Vitamin C deficiency
- Hemolysis (Excessive Destruction) of RBC's
   * Defects extrinsic to the RBC
      1. Mononuclear-phagocytic hyperactivity with hypersplenism;
      2. Immunologic disorders, a. Isoimmune (isoagglutinin) hemolysis, b. Autoimmune hemolysis, (1) Warm-antibody (Coombs-positive) hemolysis, (2) Cold-antibody hemolysis, (a) Cold aggulation, (b) Paroxysmal cold hemoglobinuria, (c). Paroxysmal nocturnal hemoglobinuria
      3. Mechanical injury a.: Trauma (with microangiopathic hemolysis) or infection.
   * Intrinsic RBC defects:
      1. Membrane alterations: a. Inborn, (1) Congenital erythropoietic porphyria, (2) Heriditary elliptocytosis, (3) Hereditary spherocytosis, b. Acquired (1) Stomatocytosis (2) Hypophosphatemia 2. Metabolic disorders (inherited enzyme deficiencies) a. Embden-Meyerhof pathway defects (G6PD deficiency) 3. Hemoglobinopathies a. Sickle cell anemia (Hb S) b. Hb C, S-C, and E diseases, c. Thalassemias (β,β-δ, and α), and d. Hb S-β-thalassemia disease.

This list is intended to be inclusive but not exhaustive.

Since anemia can arise from so many different sources, treating anemia necessarily involves first identifying the cause of the anemia, then developing a course of treatment or therapy which eliminates the cause or treats the symptoms, consequently preventing or treating CHF in a patient suffering from this disease.

### Anemias and Treatment Regimens

Bleeding can lead to acute posthemorrahagic anemia. Treatment involves stopping the bleeding followed by blood transfusion, which is the only reliable means of restoring blood volume. Hemostasis and blood transfusion technologies are well known.

Chronic post hemorrahagic anemia usually arises from slowly losing blood over a long period. Chronic GI bleeding, as occurs with some types of ulcers is a frequent cause of this type of anemia. Urological or gynecological bleeding may also be sources of chronic posthemorrhagic anemia. The cause of the hemorrhage must be identified and rectified. Thereafter treatment is similar to that of treating iron-deficiency anemia, discussed more fully below.

Defective or deficient heme or globin synthesis produces something called a hypochromic-microlytic red blood cell population. This state can arise from iron deficiency, iron-transport deficiency, disorders in iron utilization or reutilazition attributed to chronic diseases, or thalassemias.

If iron deficiency is the cause, it may have been brought about by poor iron absorption or iron metabolism. Inadequate iron in the diet can be remedied by taking iron supplements in the form of pills or elixirs. Vitamin C is known to increase the bioavailability of iron; supplemental vitamin C therapy may be useful.

Hemosiderosis, iron overload in tissue or deposition of iron in tissue, may result in anemia. If tissue injury is associated with deposition, it is called hemochromatosis. Phlebotomy can be used to remove excess iron. If pulmonary hemosiderosis is involved, oral iron may be effective if the anemia is not severe. Blood transfusions may be required. Corticosteroids and immunosuppressive drugs have been use in severe cases.

Iron-deficiency anemia is the most common cause of anemia. It is almost always caused by blood loss of some form. In men occult blood loss is the most common cause. In women it is menstration. Treatment starts with identifying the cause and rectifying it if possible. Oral iron supplements with or without vitamin C are often recommended as follow-up treatment once the cause of blood loss is rectified.

Iron utilization amenias are caused by inadequate or abnormal utilization of intercellular iron for hemoglobin synthesis. Thalassemic type diseases or iron overload anemias are usually the cause of these anemias. Treatment needs to recognize the cause of the anemia and its removal. Alcohol may be involved as a causative agent. Blood transfusions may be appropriate in some instances, especially if a cardiopulmonary event is or may be involved.

Iron reutilization anemia is the second most common form of anemia. In this form of the disease the problem lies in the fact that the bone marrow erythroid mass fails to respond to the anemia. A chronic disorder such as an infection, inflammatory disease or cancer may be a contributing factor to the failure of the erythroid mass to expand. Aside from removing the chronic disease, treatment with recombinant erythropoietin has been shown to provide a significant increase in RBC.

Decreased RBC production can arise from lack of normal humoral stimulus, be caused by loss of RBC precursors and the replacement of normal marrow cells by abnormal or nonhematopoietic cells. Hypoproliferative anemias appear to be associated with decreased production of erythropoietin. Accordingly treatment with EPO is indicated. Iron deficiencies can also cause hypoproliferation. Increased iron intake is thus warranted.

Renal disease may result in anemia. Reduced EPO production may result in inadequate humoral stimulus of RBC production. Reestablishing renal function treats this disease. Alternatively, treating a patient suffering from renal failure with EPO can increase RBC production as well.

Pernicious anemia arises from a deficiency of vitamin B₁₂. The cause my be dietary. For example, vegetarians may not consume adequate amounts of the vitamin, which is more prevalent in animal tissue that it is in vegetative dietary sources. Legumes do provide adequate levels of this vitamin. Vitamin supplements can be taken or the cobalamins can be given by injection.

Anemias arising from folic acid deficiency, copper deficiency and vitamin C deficiencies all can be treated by dietary supplements. Vitamin supplements are readily available. Changes in dietary habits can contribute to prophylactic treatment of anemias.

If the cause of the anemia is hemolysis related to intrinsic RBC defects, treatment should follow a recognized regimen. Membrane alteration-caused anemias. inherited enzyme deficiencies and hemoglobinpathies, if they are the cause of the anemia, can be treated to the extent they can be treated using currently available techniques and intervention.

In the practice of this invention, one should first determine that the patient is anemic, or that the patient may develop an anemia due to genetic predisposition or lifestyle. If anemia exist, the cause of the anemia should be treated if that is an appropriate course of action. Acute causes must be addressed immediately. If chronic, the underlying cause should be treated if possible. Then the patient should be put on a treatment regimen which will increase the RBC or hemoglobin levels, as appropriate, to about the normal range.

If a patient faces a likelihood of developing an anemia, preventative treatment or course of conduct should be instituted. For example if the the patient is diagnosed with a disease which is know to result in anemia, and that disease is treatable by, for example iron supplements or administering erythropoietin, the appropriate regimen should be instituted. This can prevent the onset of an anemic condition which could adversely impact the congestive heart failure condition of that patient. A specific treatment regimen will depend on the type of anemia identified.

Treating anemias or instituting preventive treatment is within the skill of the medical profession. Reference is made to the medical literature for the appropriate treatment for a given anemia.

## Claims

1. The use a compound which treats anemia in the manufacture of a medicament for use in treating conjestive heart failure (CHF) in patient suffering from CHF and who are anemic, excluding patients with kidney disease or dysfunction.

2. The use a compound which treats anemia in the manufacture of a medicament for use in treating conjestive heart failure (CHF) in patient suffering from CHF and who are anemic.

3. The use of a compound which treats anemia in the manufacture of a medicament for use in preventing an occurance of conjestive heart failure by preventing the onset or worsening of anemia.

4. The use of a compound which treats anemia in the manufacture of a medicament for use in preventing an occurance of conjestive heart failure by preventing the onset or worsening of anemia, excluding patients with kidney disease or dysfunction.

5. The use of a compound according to claims 1 and 4 wherein the compound is erythropoietin.

6. The use of a compound according to claims 1-4 wherein the compound is a vitamin B₁₂ supplement, a folic acid supplement; a copper supplement, or a vitamin C supplement.
